## Europäisches Patentamt

## European Patent Office

## Office européen des brevets

(19)

(11) Veröffentlichungsnummer: **0 182 248 B1**

# EUROPÄISCHE PATENTSCHRIFT

(12)

(45) Veröffentlichungstag der Patentschrift: **17.04.91**

(51) Int. Cl.⁵: **A61M 1/00**

(21) Anmeldenummer: **85114343.8**

(22) Anmeldetag: **12.11.85**

(54) **Vorrichtung zum Absaugen von Sekretflüssigkeit aus einer Wunde.**

(30) Priorität: **16.11.84 DE 3441891**

(43) Veröffentlichungstag der Anmeldung:
**28.05.86 Patentblatt 86/22**

(45) Bekanntmachung des Hinweises auf die
Patenterteilung:
**17.04.91 Patentblatt 91/16**

(84) Benannte Vertragsstaaten:
**AT BE CH DE FR GB IT LI LU NL SE**

(56) Entgegenhaltungen:
**EP-A- 0 036 546**
**EP-A- 0 109 460**
**FR-A- 2 210 416**
**FR-A- 2 527 451**
**US-A- 4 475 904**

(73) Patentinhaber: **Beck, Walter**
**Heckenweg 5**
**W-7250 Leonberg(DE)**

Patentinhaber: **Werner, Margrit, Dipl.-Ing.**
**Lion-Feuchtwanger-Strasse 69**
**W-6500 Mainz-Hechtsheim(DE)**

(72) Erfinder: **Beck, Walter**
**Heckenweg 5**
**W-7250 Leonberg(DE)**
Erfinder: **Berger, Siegfried, Dipl.-Ing.**
**Hauptstrasse 6**
**W-7314 Wernau(DE)**
Erfinder: **Werner, Margrit, Dipl.-Ing.**
**Lion-Feuchtwanger-Strasse 69**
**W-6500 Mainz-Hechtsheim(DE)**

(74) Vertreter: **Patentanwälte Phys. Bartels**
**Dipl.-Ing. Fink Dr.-Ing. Held**
**Lange Strasse 51**
**W-7000 Stuttgart 1(DE)**

# Beschreibung

Die Erfindung betrifft eine Kombinatiou einer Vorrichtung zum Absaugen von Sekretflüssigkeit aus einer Wunde mit einer Schlauchpumpe, welche die Merkmale des Oberbegriffes des Anspruches 1 aufweist.

Bei bekannten Absaugvorrichtungen (EP-A-0 036546) beginnt das Absaugen der Sekretflüssigkeit mit maximalem Unterdruck, also maximaler Saugwirkung. Entsprechend der abgesaugten Flüssigkeitsmenge nehmen der Unterdruck und damit auch die Saugwirkung ab. Bedingt ist dies dadurch, daß sich der in der Flasche herrschende Unterdruck durch die in die Flasche gesaugte Sekretflüssigkeit mehr und mehr abbaut. Störend ist bei diesen bekannten Vorrichtungen nicht nur die unter Umständen anfänglich zu hohe Saugleistung, sondern vor allem auch die fehlende Möglichkeit, die Saugleistung an die Erfordernisse anpassen und beispielsweise über einen längeren Zeitraum auch annähernd konstant halten zu können. Hinzu kommt noch, daß bei den bekannten Vorrichtungen zumindest dann eine Kontaminationsgefahr besteht, wenn ein Auswechseln der Flasche erforderlich ist, weil hierzu die Verbindung zwischen der Flasche und dem Schlauch, an den der Drain angeschlossen ist, geöffnet werden muß.

Zwar ist auch eine Absaugvorrichtung bekannt (FR-A-2 527 451), bei welcher die Flasche, an welche der zum Drain führende Schlauch angeschlossen ist und die an eine Unterdruckquelle angeschlossen ist nicht ausgetauscht zu werden braucht, weil am Boden dieser Flasche ein mittels eines Rückschlagventiles verschließbarer Auslaß vorgesehen ist, mit dem lösbar ein Beutel verbunden ist. Nachteilig ist hierbei vor allem, daß nur eine diskontinuierliche Absaugung möglich ist, weil die Abgabe der in der Flasche gesammelten Flüssigkeit an den Beutel nur möglich ist, wenn in der Flasche kein Unterdruck herrscht und deshalb das Rückschlagventil öffnet.

Weiterhin ist eine Kombination einer Vorrichtung zum Absaugen von Sekretflüssigkeit aus einer Wunde mit einer Schlauchpumpe bekannt (FR-A 2 210 416), bei der zwischen einen zum Drain führenden ersten Schlauch und einen mit der Schlauchpumpe zusammenwirkenden Abschnitt eines zweiten Schlauches die Membrandose einer Unterdruck-Anzeigevorrichtung eingefügt ist. Die ständig an den ihr zugeordneten Schlauchabschnitt angeschlossene Schlauchpumpe, die von der Unterdruck-Anzeigevorrichtung so im Wechsel aus- und eingeschaltet wird, daß ständig im ersten Schlauch ein bestimmter Unterdruck herrscht, wird von einer Grundplatte getragen, welche außer den Anschlußstutzen für den ersten Schlauch und den mit der Schlauchpumpe zusammenwirkenden Schlauchabschnitt einen Anschlußstutzen für einen Schlauchabschnitt aufweist, der von der Schlauchpumpe zu einem Beutel führt, in welchen die Schlauchpumpe die abgesaugte Sekretflüssigkeit pumpt. Nachteilig ist dieser Kombination vor allem, daß eine kontinuierliche Sekretabsaugung erfordert, daß die Schlauchpumpe ständig betriebsbereit ist. Dies hat nicht nur zur Folge, daß bei einem Ausfall der Energieversor gung kurzfristig mit anderen Mitteln die Sekretabsaugung fortgeführt werden muß. Vor allem ist der Patient weitgehend immobil, weil ständig eine Verbindung zwischen der Schlauchpumpe und dem Netz vorhanden sein muß. Eine ausreichende Mobilität ist jedoch in der postoperativen Phase in der Regel von großer Bedeutung.

Der Erfindung liegt die Aufgabe zugrunde, eine Vorrichtung zum Absaugen von Sekretflüssigkeit aus einer Wunde zu schaffen, die keine Anwendungsprobleme mit sich bringt, also nicht nur die Anpassung des wirksamen Unterdruckes an die Erfordernisse ermöglicht, sondern auch während der gesamten Drainagedauer keine Öffnung des geschlossenen Systems erforderlich macht. Diese Aufgabe löst eine Vorrichtung mit den Merkmalen des Anspruches 1.

Dadurch, daß der Unterdruck in der die abgesaugte Sekretflüssigkeit zunächst aufnehmenden Flasche von Zeit zu Zeit auf einen wählbaren Wert regeneriert werden kann, braucht der anfänglich wirksame Unterdruck nicht so gewählt zu werden, daß die Saugwirkung möglichst lange vorhanden ist. Vielmehr kann er entsprechend dem anfänglich optimalen Wert gewählt werden. Ebenso kann anschließend der Unterdruck, welcher auch bei der erfindungsgemäßen Lösung durch die in die Flasche gelangende Sekretflüssigkeit vermindert wird, zu jedem beliebigen Zeitpunkt wieder auf den gewünschten Wert gebracht werden, der gleich hoch wie der Ausgangswert, aber auch niedriger oder höher liegen kann. Ferner ist die Kontaminationsgefahr ausgeschlossen, weil die Flasche während der gesamten Dauer der Sekretflüssigkeitsabsaugung über den Schlauch mit dem Drain verbunden bleiben kann, da bei der Regeneration des Unterdruckes die Sekretflüssigkeit, die sich in der Flasche angesammelt hat, abgeführt werden kann. Da der aus der Flasche abgesaugte Inhalt derselben durch einen Schlauch hindurch in einen Sammelbehälter eingeleitet wird, indem der zum Sammelbehälter führende Schlauch der Einwirkung einer Schlauchpumpe ausgesetzt wird, ist sichergestellt, daß das System auch während der Regenerationsphase vollständig geschlossen bleibt. Weil das Absaugen des Inhaltes der Flasche und die erneute Einstellung des Unterdrucks in der Flasche nur von Zeit zu Zeit zu erfolgen braucht, ist es nicht notwendig, daß das geschlossene System ständig mit der Schlauchpumpe kombiniert ist. Vielmehr genügt es, den von der Flasche zum Sammelbehälter führenden Schlauch nur während jeder Regenerationsphase der Wirkung der Schlauchpumpe auszusetzen. Es können dann mit einer einzigen Schlauchpumpe mehrere Sekretflüsigkeitsabsaugsysteme versorgt werden, was den Aufwand erheblich vermindert. Allerdings ist zumindest dann, wenn die Schlauchpumpe abgenommeu werden kann, für den zweiten Schlauch als Absperrvorrichtung eine Schlauchklemme erforderlich, um am Ende jedes Regenerationsvorganges den zweiten Schlauch zwischen der Flasche und dem Sammelbehälter absperren zu können.

Da die erfindungsgemäße Lösung die bekannte Flasche, in der ein Unterdruck herrscht, beibehält, ist insoweit der Gebrauch der erfindungsgemäßen Lösung dem Personal in Krankenhäusern und Kliniken vertraut. Dies erleichtert bereits wesentlich die Handhabung. Hinzu kommt lediglich, daß von Zeit zu Zeit an den von der Flasche zum Sammelbehälter führenden Schlauch die Schlauchpumpe angesetzt oder die angesetzte Schlauchpumpe eingeschaltet zu werden braucht, was ebenfalls keine Schwierigkeiten bereitet. Die Handhbung gegenüber den bekannten Systemen wird sogar noch vereinfacht, weil ein Flaschen austausch nicht erforderlich ist. Sofern die Schlauchpumpe ständig mit dem zweiten Schlauch kombiniert ist und eine Ausbildung hat, die sicherstellt, daß der zweite Schlauch abgesperrt wird, kann die Schlauchpumpe selbst die Absperrvorrichtung bilden.

Vorzugsweise besteht die Unterdruck-Anzeigevorrichtung aus einem Faltenbalg, dessen Innenraum mit dem Innenraum der Flasche in Verbindung steht, einer am Faltenbalg angreifenden Rückstellfeder und einer Skala, an welcher aufgrund der Stellung des Faltenbalges oder auch der Länge der Rückstellfeder der herrschende Unterdruck abgelesen werden kann. Besonders vorteilhaft ist eine derartige Unterdruck-Anzeigevorrichtung insofern, als sie nur aus einfachen, kostengünstigen Teilen besteht. Der Faltenbalg kann dabei aus Gummi oder Kunststoff bestehen. Die Anzeigeeinrichtung kann deshalb zusammen mit der Flasche nach dem Gebrauch weggeworfen werden. Bei einem sehr großen Anzeigebereich kann es zur Vermeidung eines relativ langen Faltenbalges und einer relativ langen Rückstellfeder vorteilhafter sein, wenigstens zwei Unterdruck-Anzeigevorrichtungen dieser Art vorzusehen, die unterschiedliche Meßbereiche haben.

Aus Kostengründen ist vorzugsweise der Sammelbehälter als Beutel ausgebildet. Aus demselben Grund kann als Absperrvorrichtung eine Schlauchklemme vorgesehen sein.

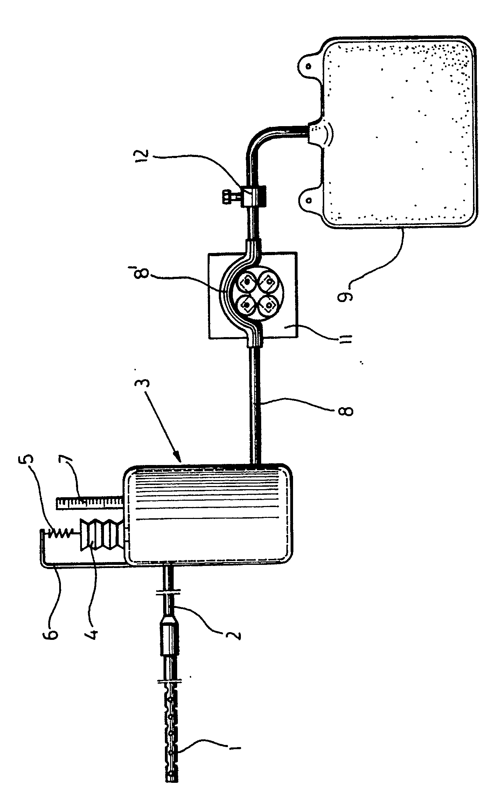

Im folgenden ist die Erfindung anhand eines in der Zeichnung dargestellten Ausführungsbeispiels der erfindungsgemäßen Vorrichtung im einzelnen erläutert. Die einzige Figur zeigt eine unvollständig dargestellte Ansicht dieser Vorrichtung.

Ein in bekannter Weise ausgebildeter Drain 1 ist über eine ersten Schlauch 2 mit einer formstabilen, aus Kunststoff bestehenden Flasche 3 verbunden. Der nicht dargestellte Stutzen der Flasche für den Drain 2 befindet sich nahe dem die Flasche 3 nach oben verschließenden Deckel. Dieser Deckel ist im Ausführungsbeispiel mit einem einzigen Anschlußstutzen versehen, mit dem dicht das eine Ende eines Faltenbalges 4 verbunden ist. Es könnte aber auch ein zweiter Anschlußstutzen für einen zweiten Faltenbalg vorhanden sein. Der Faltenbalg 4, dessen Innenraum mit dem Innenraum der Flasche 3 über den Stutzen in Verbindung steht, ist an seinem von der Flasche 3 wegweisenden Ende mit dem einen Ende einer Schraubenzugfeder 5 verbunden, deren anderes Ende an einen fest mit der Flasche 3 verbundenen Halter 6 angehängt ist. Die Feder 5 bildet eine Rückstellfeder, welche den aus Gummi oder Kunststoff bestehenden Faltenbalg 4 zu strecken sucht. Ein Unterdruck in der Flasche 3 hat zur Folge, daß sich der Faltenbalg 4 verkürzt und dabei die Feder 5 entsprechend verlängert wird. Der Faltenbalg 4 und die Feder 5 bilden also zusammen einen Sensor für die Höhe des Unterdruckes in der Flasche. Um die Größe des Unterdruckes anzuzeigen, ist neben dem Faltenbalg 4 eine Skala 7 angeordnet, an der sich die Länge des Faltenbalges 4 und die zugehörige Höhe des Unterdruckes in der Flasche 3 ablesen läßt. Die Skala 7, der Faltenbalg 4 und die Schraubenfeder 5 mit dem sie haltenden Halter 6 bilden daher zusammen eine Unterdruck-Anzeigevorrichtung.

Nahe dem Boden der Flasche 3 ist diese mit einem nicht dargestellten Stutzen versehen, auf den das eine Ende eines zweiten Schlauches 8 aufgesteckt ist. Dieser zweite Schlauch führt zu einem Sammelbehälter 9, der im Ausführungsbeispiel ein Beutel ist und zweckmäßiger Weise so gewählt ist, daß er, ohne ausgetauscht werden zu müssen, die gesamte Sekretflüssigkeitsmenge aufnehmen kann. Das System kann dadurch vollkommen geschlossen bleiben.

Der zweite Schlauch 8 weist einen aus Silikongummi bestehenden Abschnitt 8' auf, der für das Zusammenwirken mit einer Schlauchpumpe 11 bestimmt ist. Ferner trägt der zweite Schlauch 8 in dem Bereich zwischen dem Abschnitt 8' und dem Sammelbehälter 9 ein manuell zu betätigende Schlauchklemme 12.

Nachdem der Drain 1 in die Wunde eingeführt und die Wunde verschlossen worden ist, wird mittels der Schlauchpumpe 11 die Flasche 3 soweit evakuiert, bis der für die Absaugung der Wundsekretflüssigkeit optimale Unterdruck in der Flasche 3 erreicht ist. Sodann wird die Schlauchklemme 12 geschlossen und die Schlauchpumpe 11 abgeschaltet oder ganz abgenommen, damit sie anderweitig eingesetzt werden kann. Durch die zunehmende Menge an Sekretflüssigkeit, die sich in der Flasche 3 ansammelt, nimmt der Unterdruck im Inneren der Flasche 3 ab. Diese Abnahme ist an der Skala 7 ablesbar, weil die Feder 5 den Faltenbalg 4 entsprechend verlängert.

So bald der Unterdruck in der Flasche 3 den noch zulässigen unteren Grenzwert erreicht hat, wird die Schlauchpumpe 11 erneut wirksam gemacht, indem sie eingeschaltet oder an den Abschnitt 8' des Schlauches 8 angesetzt und danach eingeschaltet wird. Während die Schlauchpumpe 11 arbeitet, muß die Schlauchklemme 12 geöffnet sein, damit die aus der Flasche 3 abgepumpte Sekretflüssigkeit in den Sammelbehälter 9 fließen kann. Das Absaugen der Sekretflüssigkeit aus der Flasche 3 hat zur Folge, daß der Unterdruck in der Flasche 3 wieder ansteigt. So bald der gewünschte Wert erreicht ist, was an der Skala 7 erkennbar ist, wird die Schlauchklemme 12 wieder geschlossen und die Schlauchpumpe 11 abgeschaltet, gegebenenfalls auch vom Abschnitt 8' getrennt. Nach Beendigung der Absaugung der Sekretflüssigkeit werden alle Teile mit Ausnahme der Schlauchpumpe 11 weggeworfen.

Wie sich aus dem geschilderten Verfahrensablauf ergibt, kann man während des Absaugens der Sekretflüssigkeit den Unterdruck in der Flasche 3 und damit die Saugwirkung innerhalb beliebig wählbarer Bereiche halten. Außerdem braucht das in mikrobiologischer Hinsicht geschlossene System, bestehend aus dem Drain 1, dem ersten Schlauch 2, der Flasche 3, dem zweiten Schlauch 8 und dem Sammelbehälter 9 nicht geöffnet zu werden, so daß keinerlei Kontaminationsgefahr besteht.

**Ansprüche**

1. Kombination einer Vorrichtung zum Absaugen von Sekretflüssigkeit aus einer Wunde mit einer Schlauchpumpe, wobei
   a) die ein geschlossenes System bildende Absaugvorrichtung
      a1) einen an einen ersten Schlauch (2) angeschlossenen Drain (1),
      a2) eine mit dem ersten Schlauch (2) in Verbindung stehende und den in ihm herrschenden Unterdruck ermittelnde Unterdruck-Anzeigevorrichtung (4 - 7) und
      a3) einen den ersten Schlauch (2) mit einem Behälter (9) verbindenden zweiten Schlauch (8, 8') aufweist und
   b) der zweite Schlauch (8, 8') für das Zusammenwirken mit der Schlauchpumpe (11) einen zwischen dem ersten Schlauch (2) und dem Behälter (9) liegenden Abschnitt (8') hat,
   dadurch gekennzeichnet, daß
   c) die Absaugvorrichtung durch eine evakuierbare Flasche (3) ergänzt ist, die zwischen den ersten Schlauch (2) und den über eine Absperrvorrichtung (11, 12) absperrbaren zweiten Schlauch (8, 8') eingefügt ist,
   d) die Unterdruck-Anzeigevorrichtung (4 - 7) mit dem Innenraum der Flasche (3) in Verbindung steht und
   e) mittels der Schlauchpumpe (11) der Unterdruck in der Flasche (3) von Zeit zu Zeit auf einen wählbaren Wert regenerierbar und dabei die in der Flasche (3) angesammelte Sekretflüssigkeit in den Behälter (9) überführbar ist.

2. Kombination nach Anspruch 1, dadurch gekennzeichnet, daß am zweiten Schlauch (8, 8') zusätzlich zur Schlauchpumpe (11) als Absperrvorrichtung eine Schlauchklemme (12) vorgesehen ist, mittels deren die durch den zweiten Schlauch (8, 8') gebildete Leitung absperrbar ist.

3. Kombination nach Anspruch 2, dadurch gekennzeichnet, daß die Schlauchpumpe (11) von Zeit zu Zeit an den zweiten Schlauch (8, 8') ansetzbar ist, wobei nach dem Ansetzen der Schlauchpumpe (11) die Schlauchklemme (12) zu öffnen und vor dem Abnehmen der Schlauchpumpe (11) wieder zu schließen ist.

4. Kombination nach einem der Ansprüche 1 bis 3, dadurch gekennzeichnet, daß die Unterdruck-Anzeigevorrichtung (4 - 7) wenigstens einen Faltenbalg (4), dessen Innenraum mit dem Innenraum der Flasche (3) in Verbindung steht, eine am Faltenbalg (4) angreifende Rückstellfeder (5) und eine Skala (7) aufweist.

5. Kombination nach einem der Ansprüche 1 bis 4, dadurch gekennzeichnet, daß der Behälter (9) als Beutel ausgebildet ist.

6. Kombination nach einem der Ansprüche 1 bis 5, dadurch gekennzeichnet, daß die Absaugvorrichtung aus Einwegteilen zusammengesetzt ist.

**Claims**

1. Combination of a device for the suction of fluid secretions from a wound with a tube pump, wherein;

a) the suction device, which forms a closed system, has

a1) a drain (1) connected to a first tube (2),

a2) a vacuum pressure display (4 - 7), which is connected to the first tube (2) and displays the vacuum pressure prevailing therein and

a3) a second tube (8, 8') connecting the first tube (2) to a container (9); and

b) the second tube (8, 8') has, a section (8') lying between the first tube (2) and the container (9) for cooperating with the tube pump (11);

characterised in that

c) the suction device is extended to include a bottle (3) which can be emptied, which is inserted between the first tube (2) and the second tube (8, 8'), and which may be closed off by means of a closing device (11, 12);

d) the vacuum pressure display (4 - 7) is connected to the inner chamber of the bottle (3); and

e) by means of the tube pump (3), the vacuum pressure in the bottle (11) can periodically be regenerated to a selectable value, and in this case the fluid secretion accumulated in the bottle (3) may be transferred to the container (9).

2. Combination according to claim 1, characterised in that on the second tube (8, 8') there is provided, in addition to the tube pump (11), a tube clamp (12) as a closing device, by means of which the channel formed by the second tube (8, 8') may be closed.

3. Combination according to claim 2, characterised in that the tube pump (11) can periodically be attached to the second tube (8, 8') wherein the tube clamp (12) is to be opened after the tube pump (11) has been attached and reclosed before the tube pump (11) is detached.

4. Combination according to one of claims 1 to 3, characterised in that the vacuum pressure display (4 - 7) is equipped with at least one set of bellows (4) the inner chamber of which connects with the inner chamber of the bottle (3), a resetting spring (5) which engages on the set of bellows (4), and a graduated scale (7).

5. Combination according to one of claims 1 to 4, characterised in that the container (9) is formed as a bag.

6. Combination according to one of claims 1 to 5, characterised in that the suction device is composed of disposable components.

## Revendications

1. Combinaison d'un dispositif d'aspiration de liquide de sécrétion à partir d'une plaie avec une pompe péristaltique, où

a) le dispositif d'aspiration-formant un système fermé comporte :

a1) un drain (1) relié à un premier tuyau souple (2),

a2) un dispositif indicateur de dépression (4-7) en liaison avec le premier tuyau souple (2) et déterminant la dépression régnant dans celui-ci et

a3) un second tuyau souple (8, 8') reliant le premier tuyau souple (2) à un récipient (9), et

b) le second tuyau souple (8, 8') comporte, pour la coopération avec la pompe péristaltique (11), un tronçon (8') situé entre le premier tuyau souple (2) et le récipient (9),

- caractérisée en ce que :

c) le dispositif d'aspiration est complété par une bouteille (3) pouvant être mise sous vide et qui est interposée entre le premier tuyau souple (2) et le second tuyau souple (8, 8') pouvant être obturé par l'intermédiaire d'un dispositif de fermeture (11, 12),

d) le dispositif indicateur de dépression (4 - 7) est relié au volume intérieur de la bouteille (3) et

e) au moyen de la pompe péristaltique (11), la dépression dans la bouteille (3) peut être rétablie de temps à autre à une valeur sélectionnable et alors le liquide de sécrétion collecté dans la bouteille (3) peut être transféré dans le récipient (9).

2. Combinaison selon la revendication 1, caractérisée en ce que dans le second tuyau souple (8, 8'), il est prévu en addition à la pompe péristaltique (11), une pince à tuyau souple servant de dispositif de fermeture (12), au moyen duquel peut être fermé le conduit constitué par le second tuyau souple (8, 8').

3. Combinaison selon la revendication 2, caractérisée en ce que la pompe péristaltique (11) peut être reliée de temps à autre au second tuyau souple (8, 8'), auquel cas il faut ouvrir la pince à tuyau souple (12) après l'installation de la pompe péristaltique (11) et refermer cette pince après l'enlèvement de la pompe péristaltique (11).

4. Combinaison selon une des revendications 1 à 3, caractérisée en ce que le dispositif indicateur de dépression (4 - 7) comporte au moins un soufflet plissé (4), dont le volume intérieur est relié au volume intérieur de la bouteille (3), un ressort de rappel (5) agissant sur le soufflet (4) et une échelle (7).

5. Combinaison selon une des revendications 1 à 4, caractérisée en ce que le récipient (9) est agencé comme une vessie.

6. Combinaison selon une des revendications 1 à 5, caractérisée en ce que le dispositif d'aspiration est composé de parties jetables après usage.